# EUROPEAN PATENT APPLICATION

(11) **EP 1 944 281 A1**
(43) Date of publication of application: **16.07.2008**
(21) Application number: 07100290.1
(22) Date of filing: 09.01.2007
(51) Int. Cl.: C07B 59/00, C07C 25/13, A61K 51/00

(54) **18F-labeled compounds, method for the preparation and use thereof**

(71) Applicant: Technische Universität München, 80333 München (DE)
(72) Inventor: Henriksen, Gjermund, 80636, München (DE); Hooshyar Yousefi, Behrooz, 81738, München (DE); Wester, Hans-Jürgen, 85304, Ilmmünster (DE)
(74) Representative: Behnisch, Werner

(57) **Abstract**

The present invention relates to method for the preparation of a ¹⁸F-labeled aniline derivative of the general formula I 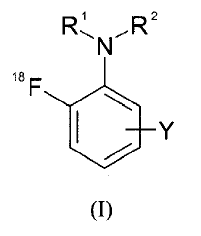 by fluorination of an aniline derivative with [¹⁸F]fluoride under the application of microwave heating, wherein X is a leaving group; R¹ and R², independently of one another, are H, alkyl, alkenyl, alkinyl, aryl, heteroaryl and alkylaryl, and wherein R¹ and R² can be joined together to form a ring structure; and Y represents one or more substituents selected from the group consisting of H, alkyl, alkenyl, alkinyl, aryl, heteroaryl and alkylaryl, halogen, fused aryl and fused heteroaryl. Further, the invention relates to ¹⁸F-labeled compounds of the above given formula and the use thereof in non-invasive imaging of a mammal, preferably a human, by positron emission tomography, especially for obtaining information on the extend, kinetics or treatment response of a disease related to pathological proteins in the central nervous system.

## Description

The present application relates to ¹⁸F-labeled compounds, a method for the preparation of these compounds and the use thereof in diagnostics.

### General background of the invention

Alzheimer's disease (AD) is a neurodegenerative disease characterized by dementia, cognitive impairment, and memory loss. Postmortem brains of AD patients reveal neuropathological features: the presence of senile plaques (SPs) and neurofibrillary tangles (NFTs), which contain β-amyloid (Aβ) peptides and highly phosphorylated tau proteins. Although the precise mechanism of neuronal death in AD is still unknown, it is widely accepted that SPs and NFTs play a central role in its development. Recent development to reduce the burden of amyloid-beta (Aβ) present novel opportunities to test the amyloid hypothesis in clinical trials of these therapies, but this requires reliable and sensitive methods to monitor plaque burden in the brains of living patients with Alzheimer's disease (AD). Although detection of Aβ can be done by staining postmortem brain tissue with fluorescent agents, these techniques are not applicable to monitor SP's in living patients. For the depiction and quantification *in vivo* with molecular imaging agents further demands to a tracer are encountered, hereunder a high penetrability of the blood-brain-barrier (BBB), low unspecific binding and relevant in vivo stability. Thus, quantitative evaluation of SPs and/or NFTs in the brain with noninvasive techniques such as positron emission tomography (PET) and single photon emission computed tomography (SPECT) would allow presymptomatic identification of patients and monitoring of putative neuroprotective effects of novel treatments that are currently being investigated.

Since the mid 90's, a few groups worldwide have worked on development of radiolabeled imaging agents specific for amyloid plaque. Initial efforts were impeded by poor brain entry of the tracer, high levels of non-specific binding, or low binding in regions known to contain high concentrations of Aβ.

Compounds like Chrysamine G (CG) and Congo Red (CR) possess high affinity for Aβ fibrils and have been used in fluorescent staining of plaques in postmortem brain sections. Because ionic and bulky groups are not able to penetrate intact BBB, attempts to develop radiolabeled CG and CR as imaging agents were in general unsuccessful. Other, more chemically distant analogues of CG composed of styrylbenzene salicylic acids, such as X34, provided similar results, i.e. high in vitro binding affinity to aggregated Aβ and low brain penetration in normal rodent brain.

Clinical trials in AD patients have been reported with several agents including [¹⁸F]2-(1-(2-(*N-*(2-fluoroethyl)-*N*-methylamino)naphthalene-6-yl)ethylidene)malononitrile ([¹⁸F]FDDNP), [¹¹C]-2-(4-(methylamino)-phenyl)-6-hydroxybenzothiazole ([¹¹C]6-OH-BTA-1) and [¹¹C]-4-*N-*methylamino-4'-hydroxystilbene ([¹¹C]SB-13). In addition, several compounds belonging to the BTA, stilbene and IMPY series have been described as Aβ-affine probes. In addition, benzofuran derivatives, designed to be isosteric analogues of thioflavin-T have been described (see Fig. 1).

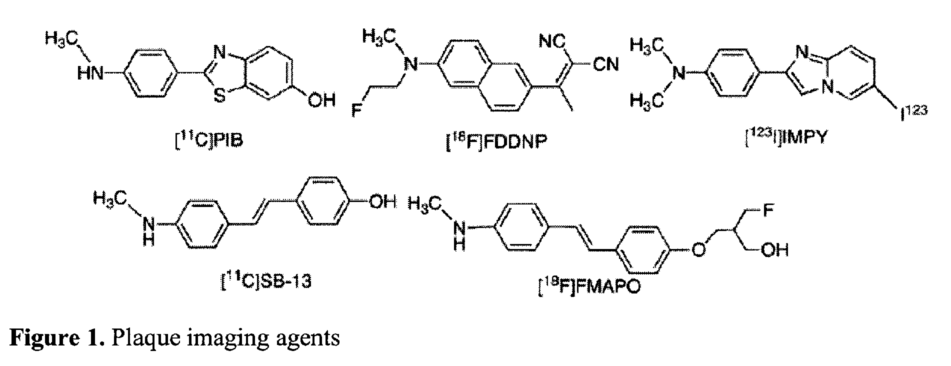

In the following, three different classes of compounds with affinity to Aβ are presented in more detail.

### Compound Class 1: Benzothiazoles

Radioiodinated derivatives of thioflavin have been shown to have a potential with their high binding affinities for aggregated Aβ and good penetration in mouse brain (Zhuang et al. 2001, 2003). Recently an *in vitro* and *in vivo* study of a series of neutral Thioflavin-T derivatives; 2-arylbenzothiazoles, was reported (Mathis et al., 2003). Especially the compound *N*-[¹¹C-*methyl*]-2-(4'-methylaminophenyl)-6-hydroxybenzothiazol ([¹¹C]6-OH-BTA-1) was shown to have a high and rapid uptake in brain, high retention in amyloid-rich regions as well as rapid clearance from normal mouse brain. The first PET-studies of AD-patients revealed a selective retention of [¹¹C]6-OH-BTA-1 in amyloid-rich regions.

### Compound Class 2: IMPYs

In an effort to develop ¹²³I-labeled tracers suitable for SPECT, a series of radioiodinated probes based on various core structures have been developed resulting in the lead strucuture 6-iodo-2-(4'-dimethylamino-)phenylimidazo[1,2-a]pyridine (IMPY).

A study of a ¹⁸F-fluorinated phenylimidazo[1,2-a] pyridine derivatives (i.e. fluorinated analogues of IMPY) show that also these ligands are very rapidly metabolized. The authors resume the importance of development of ligands having higher in vivo stability and affinity as well as a lower lipophilicity (Cai et al., 2004). Based on the character of the metabolites, metabolic conversion at the anilino-nitrogen was found likely.

### Compound Class 3: Styrenes

A novel series of ¹⁸F-labeled polyethyleneglycol (PEG)-stilbene derivatives as potential Aβ-plaque specific imaging agents has been described.

As they are contained in numerous compounds that have been demonstrated be useful for imaging of pathological proteins, a method for preparation of ¹⁸F-labeled-anilines has great commercial potential. ¹⁸F-labeled anilines may be of utility in non-invasive imaging with PET in order to depict and quantify targets that are of relevancy for diagnosis, therapy planning and therapy monitoring of neurodegenerative disorders.

The most prominent example of the success of ¹⁸F-labeled radiopharmaceuticals is 2-[¹⁸F]fluoro-2-dexoy-D-glucose ([¹⁸F]FDG) which has been established for diagnosis and therapy monitoring of several human disorders.

¹⁸F-fluoride is one of the most useful PET radionuclides currently being used in clinical nuclear medicine diagnosis. With regard to the nuclear properties, ¹⁸F has a relatively pure (97 % probability) decay mode through the emission of a positron (β⁺) of which the E_{*β*+max} of 633.5 keV is the smallest of the radionuclides that are commonly used in PET. Consequently, the average range of the positron from ¹⁸F in biological tissues of about 0.3 mm is less than the inherent resolution of clinical PET-scanners. The 109.7 min half-life of ¹⁸F allows for the production and distribution of radiotracers to nuclear medicine facilities without an on-site cyclotron facility and scanning procedures extending several hours.

¹⁸F-fluoride is commonly available from cyclotrons on a Ci-scale (1 Ci = 37GBq). Among the four main production routes that have been described in the literature (for review see Wester 2003), the ¹⁸O(p,n)¹⁸F nuclear reaction on highly enriched H₂[¹⁸O]O is the most effective one, and gives ¹⁸F-fluoride in high specific activities (~5000 mCi/µmol) suitable for nucleophilic reactions of a variety of substrates.

¹⁸F-labeled compounds can be used in positron emission tomography (PET) to allow for the non-invasive imaging of, inter alia, biological structures, particularly those of mammals. This can involve depiction and quantification of receptors and transporters. To be generally useful, a radiopharmaceutical based on a short lived radionuclide such as ¹⁸F needs to be obtainable rapidly in high radiochemical yield and with high specific radioactivity. ¹⁸F has relatively short physical half life of 109.7 min, which means that any process for the manufacture of ¹⁸F-labeled compounds needs to occur in a short time period and has to be capable of providing the desired yields and specific activity of the compounds in question.

The literature comprises many different ways of forming ¹⁸F-labeled compounds although many of these processes involve the use of a precursor for radiolabeling activated for nucleophilic attack. The literature comprises some ways of preparing ¹⁸F-labeled compounds by electrophilic substitution. For preparing ¹⁸F-labeled compounds in a high specific radioactivity (>100 mCi/µmol; >3,7GBq/µmol), this method is hampered by the necessity of introducing carrier fluorine.

The vast majority of fluorination syntheses described in the literature concern the formation of non radioactive species and the chemistry involved is therefore not applicable as outlined below. The process requirements for the preparation of non-radioactive fluorine compounds are undemanding compared to synthesis of radioactive compounds. Where, however, ¹⁸F-labeled aromatics are to be synthesized, the syntheses routes are often much more complicated and the synthetic chemist is faced with a number of major challenges.

High radiochemical yield is obviously desirable in the same way that high yield is desirable in any organic synthesis. If more ¹⁸F is incorporated into the final product then the amount of active compound formed is higher and there is less wasted expensive starting material.

High specific radioactivity is an important issue in PET studies where the amount of tracer should be minimized to keep the biological system unperturbed. Especially for compounds intended for use in quantifying receptors in the central nervous system (CNS), which is often present at a relatively low concentration, these requirements are often limiting. Thus, these requirements impose significant limitations on the chemical-synthetical procedures used for the preparation of the ¹⁸F-labeled compounds relative to non-radioactive compounds.

A further problem faced during the synthesis of ¹⁸F labeled compounds is the possibility of side reactions. In conventional non radioactive synthesis, stoichiometric amounts of reactants would be used. This is often not possible in ¹⁸F-compound synthesis and there is typically considerable molar excess of reagents and reactants than the ¹⁸F-labeled species. The difference in the molar ratio of the ¹⁸F-containing reagent relative to other reagents present in the reaction mixture (including the molecule or species to be radiolabeled, called the precursor for radiolabeling) can represent an important difference to procedures in which the stoichiometry of reagents is in the range normally encountered in the synthesis of non-radioactive compounds. In particular, due to the relatively low amount of ¹⁸F-containing reactant present, impurities in the reaction mixture can be an important loss of yield if they possess a comparable or higher reactivity than the precursor for radiolabeling. Thus, side-reactions that are of low importance for a high yield of a given product in a non-radioactive synthesis can be a limiting factor for ¹⁸F-radiolabeling reactions.

A further issue with ¹⁸F syntheses is the potential exposure of the chemist to radiation. ¹⁸F syntheses of radiopharmaceuticals for use in PET are typically performed in lead shielded boxes (called hot-cells) and the manipulation of reagents, reactants and equipment is performed via a remote controlled apparatus (called the syntheses module). Under such conditions, the number of synthetic steps, the overall length of the procedure performed with the radioactivity present greatly affects the performance, including reliability and reproducibility, of the procedure. It is therefore advantageous to have as few steps in the procedure as possible, with the highest possible radiochemical yield in order to ensure a high performance of the radiosynthesis and to reduce the complexity of the apparatus required.

In nucleophilic fluorination starting with no carrier added, [¹⁸F]fluoride is the sole suitable approach for preparing high specific activity ¹⁸F-labeled compounds. Due to its high charge density, the fluoride ion is highly solvated in aqueous solution and a relatively poor nucleophile. As a strong base, it is protonated in the presence of acidic protons to form H[¹⁸F]F. Consequently, several major aspects have to be considered in order to reach a nucleophilic ¹⁸F-fluorination that is suitable for preparative procedure in radiophannaceutical chemistry. Firstly, the fluoride ion has to be activated in order to achieve a useful nucleophilicity and solubility. Often dipolar aprotic solvents are used as the reaction medium in order to avoid protonation of the fluoride. Due to the low molar concentration of reactive ¹⁸F-fluoride encountered at labeling reactions designed for obtaining a high specific activity of the product, potential undesired processes such as absorption losses, chemical side reactions and pseudo-carrier effects and presence of water implies that high chemical purity of chemicals, precursors and solvents are required to obtain a relevant radiochemical yield.

Radiofluorination of a nitro-moiety on an activated aromatic ring with anhydrous [¹⁸F]fluoride has also been reported. However, most aniline containing drugs are not activated with electron withdrawing groups, such as nitro, aldehyde, ketone, ester or others; therefore this reaction is not applicable for a large number of compounds. In the fields of radiopharmaceutical chemistry and radiopharmacy there is a need for development of new methods to perform radiofluorinations of aromatic compounds less or non-activated by electron withdrawing substituents.

An object of the present invention is to provide a fast method for less activated aromatic substrates, i.e. the preparation of ¹⁸F-labeled aniline derivatives wherein the ¹⁸F is introduced in a single step.

Another object of the present invention is to provide ¹⁸F-labeled aniline derivatives which can be used for PET diagnostics.

Still another object of the present invention is to provide a diagnostic method for the diagnosis of neurodegenerative disorders, by targeting molecular structures, such as proteins, characteristically related with these diseases.

The objects of the present invention are achieved by the independent claims. Preferred embodiments are set forth in the dependent claims.

### Summary of the invention

In a first aspect, the present invention relates to a method for the preparation of a ¹⁸F-labeled aniline derivative of the general formula I 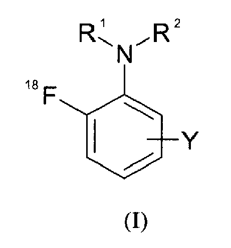 by fluorinating an aniline derivative of the general formula II 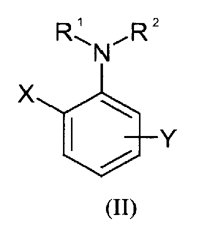 with a [¹⁸F]fluoride under the application of microwave heating,
wherein
X is a leaving group;
R¹ and R², independently of one another, are H, alkyl, alkenyl, alkinyl, aryl, heteroaryl and alkylaryl, and wherein R¹ and R² can be joined together to form a ring structure; and
Y represents one or more substituents selected from the group consisting of H, alkyl, alkenyl, alkinyl, aryl, heteroaryl and alkylaryl, halogen, fused aryl and fused heteroaryl.

It has been surprisingly found that microwave assisted radiofluorination of anilines with anhydrous tetraalkylammonium ¹⁸F-fluoride lead to ¹⁸F-labeled compounds. Said structures can be prepared in high yield and rapidly by the conversion of a suitably substituted aniline to a ¹⁸F-aniline.

In the above given aniline derivative according to formula (II), X may be any suitable leaving group for the substitution reaction by fluorine. In a preferred embodiment X is selected from the group consisting of halogen atoms, tosylsulfonate, trifluoromethanesulfonate, tolylsulfonatetriflate, tosylate, trialkyl ammonium, nitro and azide. Preferred halogen atoms are chlorine, bromine and iodine.

In a preferred embodiment of the present invention, the fluorination reaction is carried out in a solvent. The solvent is preferably a dry solvent in order to prevent the protonation of the fluoride anion. Aprotic dipolar or aprotic polar solvents are preferred as these solvent allow more easily for the solution of ionic atoms and molecules, especially the fluoride anion. Examples of solvents which may be used in the present application include dimethylformamide (DMF), tetrahydrofurane, (THF), dimethylsulfoxide (DMSO), acetonitrile, acetamide and dimethylacetamide (DMAA). However, a person skilled in the art will be able to select a suitable solvent.

The reaction according to the present invention is assisted by microwave heating. The application of microwave heating surprisingly allows for the preparation of the ¹⁸F-labeled aniline derivatives according to the present invention. The microwave heating is applied to achieve a temperature within the reaction vessel in the range of ambient temperature to 300°C, preferably in the range of 150-200°C, more preferably in the range of 160-190°C and most preferably in the range of 175-185°C. The microwave heating at a frequency of 2.45 GHz is usually applied during the whole reaction time. The power of the microwave heating is usually in a range of up to 1000 W, preferably in a range of 10 to 500 W, more preferably in a range of 50 to 300 W and most preferably in a range of 100 to 150 W. This power is applied to a reaction volume of 0.2 to 20 ml, preferably 0.5 to 5 ml.

The present invention relates to a method for fluorinating an aniline derivative. This fluorination can be applied to ¹⁸F-fluoride or any other fluoride isotope. The reaction itself does not differentiate between different isotopes. As fluoride is always present within any solvent, the concentration of the ¹⁸F-fluoride of interest is measured by it's radioactive activity. When expressing the molar ratio of the radioactive fluoride in relation to the aniline derivative, the molar ratio of the ¹⁸F-fluoride to the aniline derivative is in the range of 1:1,000-1,000,000, preferably in the range of 1:1,000-500,000.

In another preferred embodiment of the present invention, R¹ and R² are, independently of one another, substituted or unsubstituted, linear, branched or cyclic alkyl, alkenyl or alkinyl, optionally interrupted by one or more heteroatoms, preferably substituted or unsubstituted, linear or branched C₁-C₈ alkyl, C₂-C₈ alkenyl and C₂-C₈ alkinyl, optionally interrupted by one or more heteroatoms, more preferably substituted or unsubstituted, linear or branched C₁-C₆ alkyl, C₂-C₆ alkenyl and C₂-C₆ alkinyl, optionally interrupted by one or more heteroatoms, and most preferably substituted or unsubstituted, linear or branched C₁-C₄ alkyl, C₂-C₄ alkenyl and C₂-C₄ alkinyl, optionally interrupted by one or more heteroatoms. R¹ and R² may also be joined together to form a ring structure containing the nitrogen atom. The most preferred ring size contains 5 or 6 ring atoms. The most preferred substituents for R¹ and R² are H, methyl or ethyl.

In still another embodiment of the present invention, Y may be any of the above given residues in relation to R¹ and R². Further, Y may be chlorine, bromine or iodine. Another preferred substituent for Y is given by the formulae in scheme 1 below, wherein Y' is defined in the same manner as Y. 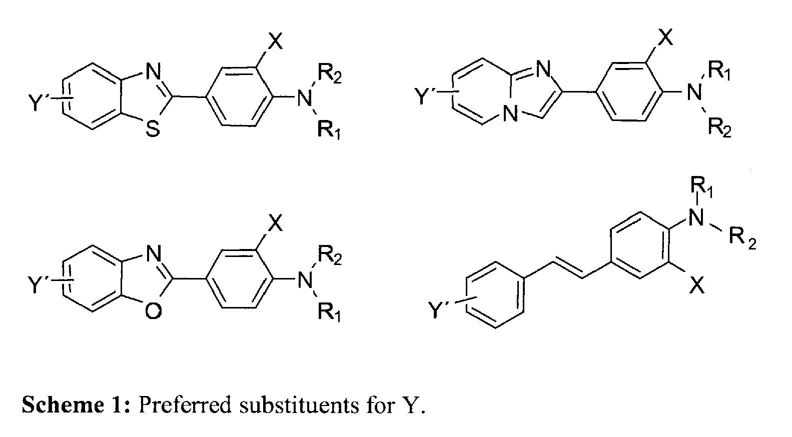 Y may be present up to four times within the compound according to formula I or II, i.e. the aniline may be substituted by one, two, three or four residues Y. Further, the aniline may be part of a fused ring system, i.e. Y is an aromatic system which is fused to the aniline ring.

A second aspect of the present invention relates to compounds of the general formula I, wherein X, R¹ and R² are defined as above and Y represents one or more substituents selected from the group consisting of H, alkyl, alkenyl, alkinyl, aryl, heteroaryl, alkylaryl, alkenylaryl, halogen, fused aryl and fused heteroaryl. Preferably, the heteroaryl substituent contains only N and/or O as hetero atoms. In another preferred embodiment, the heteroaryl substituent does not contain a benzoxazole, benzothiazole, benzimidazole and/or indole fragment.

According to a third aspect of the invention, the invention relates to the use of aniline derivatives according to formula I in non-invasive imaging by means of positron emission tomography (PET) and to the use of such compounds in clinical studies in humans and other mammals.

More preferably, the present invention relates to the use of ¹⁸F-labeled compounds in the imaging of pathological proteins in the central nervous system. The production and presence of one or more pathological proteins, especially amyloid plaques, is encountered in several human diseases and syndromes, including Alzheimer's disease, mild cognitive impairment syndrome, Parkinson's disease, Lewy body dementia, Down's syndrome, frontotemporal degeneration. The compounds of the present invention, especially the compounds of formula I, may be used to obtain information on the extend, kinetics or treatment response of a disease selected from the group consisting of Alzheimer's disease, mild cognitive impairment syndrome, Parkinson's disease, Lewy body dementia, Down's syndrome and/or frontotemporal degeneration. Imaging of these disorders using the aforementioned compounds of the general formula I provide a tool for visualization, localization and quantifiacation of the molecular structures of relevance and thus allows for obtaining information on the kinetics, progression and extent of the diseases including the evaluation of treatment responses.

### Detailed description of the invention

In recent years, the use of microwave dielectric heating to assist chemical processes has attracted increasing attention in organic, medicinal and radiochemistry community and the application of the microwave-assisted protocol has been shown to significantly enhance the speed of reactions. In particular, the use of dedicated microwave reactors that enable the rapid and safe heating of reaction mixtures in sealed vessels, under controlled conditions with on-line temperature and pressure monitoring, has greatly increased the general acceptance of the microwave heating method. In this invention, the labeling reactions were done under microwave conditions led to desired fluorinated products with the advantage of quick in core heating of dipolar and ionic spices, accelerating the achievement of high temperature and resulting improved yields.

It has now been surprisingly found that microwave assisted radiofluorinations of anilines with anhydrous tetraalkylammonium ¹⁸F-fluoride lead to ¹⁸F-labeled compound of the key (*N*-alkyl)aniline functions contained in IMPY, Styrene, BTA and Stilbenes. Said structures can be prepared in high yield and rapidly by the conversion of a suitably substituted aniline to a [¹⁸F]fluoro-aniline.

Viewed from another aspect, the invention provides the use of a ¹⁸F-labeled aromatic compound prepared by the process of the invention in the manufacture of a medicament /radiopharmaceutical for use in a method of diagnosis or treatment, such as a method for investigation of the status of receptors in the CNS. Investigation of pathological protein(s) may allow the diagnosis or treatment of diseases, such as those of the central nervous system. Specific diseases/conditions of neurodegenerative diseases such as Parkinson's and Alzheimer's disease and prion-mediated disease may be analysed by the aniline derivatives according to the present invention.

As used herein a ¹⁸F-labeled aromatic compound is a compound comprising the linkage -C-¹⁸F where C is a *sp²* hybridized carbon atom. The group X is a leaving group. The skilled chemist is aware of numerous leaving groups regularly used in organic synthesis and any of these may be used here. Preferred leaving groups are listed in standard text books such as Jerry March, Advanced Organic Chemistry and include alkoxys, halides and sulphonate esters. Preferred halides are chloride, bromide and iodide. Preferred sulphonate esters are brosylate, nosylate, mesylate, nonaflate, tresylate, triflate or tosylate, especially triflate, tosylate or mesylate. Halides and sulphonate esters are preferred in particular sulphonate esters of low molecular mass. Preferably therefore X is iodide or triflate, especially triflate (trifluoromethane sulphonyl CF₃SO₂O).

The leaving group X is preferably bound to a *sp²* c hybridized carbon atom being a part of a molecule containing the group N-R¹,R², where R¹ and R² are an alkyl group, alkylene group or hydrogen. Thus, R can also contain many other functional groups such as aryl, cycloalkyl, heterocyclic groups as well as functionalities such as hydroxyl, and other non nucleophilic and non electrophilic groups. R can therefore vary considerably as long as other functional groups present therein do not interfere with the desired C-¹⁸F formation reaction.

Yields of greater than 50%, e.g. greater than 60% can also be achieved. The reactions described herein are preferably effected with no carrier added ¹⁸F.

The amine on which this reaction is carried out can vary greatly but it needs to be a primary, secondary or tertiary amine. Ideally, the amine will be free of other functional groups which would interfere with Cₛₚ₂-¹⁸F formation, e.g. other highly nucleophilic functionalities unless protecting groups are introduced on such functionalities. The amine can however comprise all manner of organic structures such as alkyl, alkenyl, alkynyl, aryl, heterocycles, and groups such as esters, epoxides, ethers, halides etc. The person skilled in the art will appreciate that this technique is broadly applicable and the nature of the amine on which the reaction is carried out is not critical as long as the reactive amine group is primary, secondary or tertiary.

The term halogen whenever used in this application includes fluorine, chlorine, bromine and iodine.

The term alkyl as used within this application includes any linear, branched or cyclic saturated hydrocarbon. Preferably, the alkyl is a C₁ to C₁₀ alkyl, more preferably a C₁ to C₈ alkyl and even more preferably a C₁ to C₆ alkyl. However, this carbon chain may optionally be substituted or interrupted by heteroatoms like N, O and S. Preferred are interruptions by up to four heteroatoms within the carbon chain. Examples of these interruptions include ethers, thioethers, amines and polyethylene glycols (PEGs). Preferred aliphatic chains with heteroatom interruptions include PEG with 2 to 6 PEG groups and their corresponding polythioether equivalents. In another preferred embodiment, the PEG substituents contain a halogen substitution, preferably a terminal halogen substitution.

Optionally, the alkyl may further be substituted. Substitution include halogen atoms, groups containing heteroatoms like OR, NR₂, SR, SCN, CN, COOR, wherein R is H, alkyl, alkenyl, alkinyl or aryl.

The term alkenyl as used within this application is any structure as defined above for alkyl having at least one carbon carbon double bond. Similarly, the term alkinyl encompasses structions containing at least one carbon carbon triple bond.

The term aryl as used within this application includes any aromatic hydrocarbon structure. Examples of aromatic structures are phenyl, naphtyl and anthracenyl. Different aromatic rings may be fused as in the naphtyl or anthracenyl moieties. If a heteroatom is present in the ring structure it is termed as hetaryl or heteroaryl.

The term alkaryl or alkylaryl refers to an alkyl group with an aryl substituent. The term aralkyl or arylalkyl refers to an aryl group with an alkyl substituent.

The ¹⁸F-fluoride is present in the reaction as an anion. In order to bring this ¹⁸F-fluoride into a solution, a corresponding cation has also to be present. This cation is preferably a phase transfer catalyst. Any known phase transfer catalyst can be used. Preferred cationic phase transfer catalysts are tetraalkyl ammonium salts.

In an embodiment of the present application, a tetrabutyl ammonium ¹⁸F-fluoride can be used. Other ammonium salts include tetramethyl ammonium ¹⁸F-fluoride, tetraethyl ammonium fluoride, tetra(n-propyl) ammonium fluoride and tetra(iso-propyl) ammonium ¹⁸F-fluoride. The tetrabutyl ammonium ¹⁸F-fluoride may be any tetrabutyl ammonium ¹⁸F-fluoride, i.e. the butyl may be n-butyl, iso-butyl or tert.-butyl. It will be obvious to a person skilled in the art that any tetraalkyl ammonium salt may be used. This encompasses also ammonium salts wherein different alkyl radicals are present, e.g. dimethyldiethyl ammonium ¹⁸F-fluoride. However, other tetraalkyl ammonium salts may also be used, including salts wherein the alkyl groups are linear, branched or cyclic alkyl radicals. The above given definition of alkyl may also apply to the alkyl radicals of the tetraalkyl ammonium salts.

Other feasible cationic phase transfer reagents include cryptates. Thus, the cryptand [2.2.2], i.e. N[CH₂CH₂OCH₂CH₂OCH₂CH₂]₃N, is used in combination with potassium carbonate to form the cryptate [K/2.2.2]⁺.

The solution containing the ¹⁸F-fluoride may preferably contain further anions to stabilize the solution, i.e. in order to prevent the ¹⁸F-fluoride from being protonated and to form volatile H¹⁸F. The protonated form HF of the ¹⁸F-fluoride can not be used for the method according to the present application. Thus, the solution of the ¹⁸F-fluoride can also contains anionic species of carbonates or oxalates. Several pH stabilizing anions may also be used at the same time.

### Examples

### General Description

### General description of the reaction conditions

In a microwave reaction vial (size 0.5-2 ml) fitted with a magnet stirring bar, a solution of 0.5-1.5 mg of the starting *ortho*-bromo anilines in 250 µL of dry acetonitrile was introduced, followed by the addition of 50 µL of an anhydrous solution of [¹⁸F]TBAF in acetonitrile. To the reaction solution, 500 µL of dry acetonitrile was added. The microwave vial was crimped with a Teflon septum, and thereafter purged with argon for 5 min. The resulting reaction vial was kept in the microwave cavity of a microwave system (Initiator EXP, Biotage, Sweden), and heated for 30 min at 180°C. The reaction was quenched by cooling the vial with compressed air, after which the contents of the vial were subjected to chromatographic analysis (HPLC with RP18e Chromolith column, internal diameter 4.6 mm; length of 100 mm) eluted with a mixture of acetonitrile/water (27.5 : 72.5% ^{v}/ᵥ).

### Example 1: Model Compounds and Examples of the General Method

Labeling reactions were carried out in acetonitrile in the presence of a tetrabutylammonium salt (TBA⁺) and [¹⁸F]fluoride. Various leaving groups were evaluated. Reaction times were 20-40 minutes, and the reaction temperature was in the range of 150°C-200°C to result in yields in the range of 7-68% at. The highest yields of 61 ± 5 % resulted from the use of a *ortho*-bromo-leaving group and a combination of a reaction temperature of 180°C and a reaction time of 30 min. No radioactive by-product except for the desired *ortho-*[¹⁸F]fluorinated aniline derivative has been observed as measured by analytical HPLC. The reactions were according to the general reaction scheme of Scheme 2 below and are summarized in Table 1.

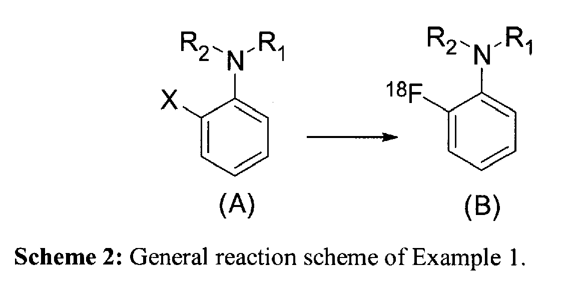

**Table 1: Summary of Examples according Scheme 2.**

| X | R₁ | R₂ | Temperature (°C) | Reaction time (min) | Yield^{a} % |
|---|---|---|---|---|---|
| I | CH₃ | CH₃ | 180 | 30 | 7 |
| Cl | CH₃ | CH₃ | 180 | 20 | 12 |
| Cl | CH₃ | H | 180 | 30 | 22 |
| Br | CH₃ | CH₃ | 150 | 30 | 27 |
| Br | CH₃ | H | 160 | 30 | 35 |
| Br | CH₃ | H | 180 | 20 | 23 |
| Br | CH₃ | H | 180 | 20 | 48 |
| Br | CH₃ | H | 180 | 30 | 61 |
| Br | CH₃ | CH₃ | 180 | 40 | 58 |

| | | | | | |
|---|---|---|---|---|---|
| ^{a} As measured by Radio-HPLC. | | | | | |

### Example 2.

Several 2-(3'-halo-4'-methylaminophenyl)-benzothiazoles were prepared according to the general method above. The reaction scheme is given in Scheme 3 below, and the reaction conditions, alkylation patterns, leaving groups and yields of the ¹⁸F-fluorination are summarized in Table 2.

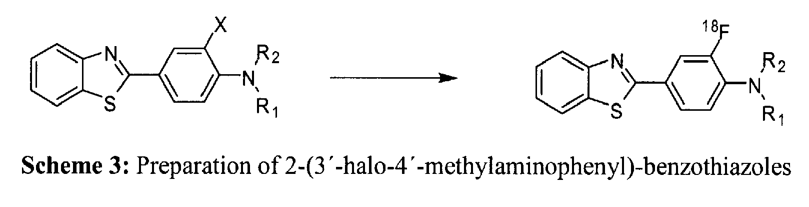

**Table 2: Summary of Examples according to Scheme 3.**

| X | R₁ | R₂ | Temperature (°C) | Reaction time (min) | Yield^{a} % |
|---|---|---|---|---|---|
| Cl | CH₃ | H | 180 | 30 | 12 |
| Cl | CH₃ | H | 180 | 30 | 14 |
| Br | CH₃ | H | 150 | 30 | 45 |
| Br | CH₃ | H | 180 | 30 | 65 |
| Br | CH₃ | CH₃ | 180 | 30 | 63 |

| | | | | | |
|---|---|---|---|---|---|
| ^{a} As measured by Radio-HPLC. | | | | | |

### Example 3:

Reaction conditions and yields of the ¹⁸F-fluorination of 6-iodo-2-(3'-bromo-4'-dimethylamino-)phenyl-imidazo[1,2-a]pyridine according to the general method.

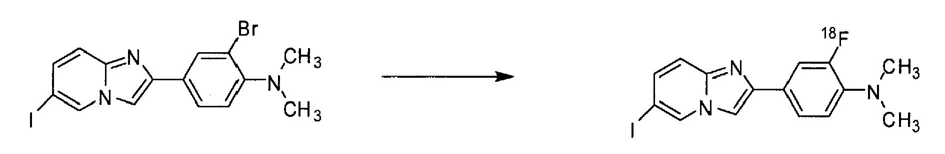

| R₁ | R₂ | Temperature (°C) | Reaction time (min) | Yield^{a} % |
|---|---|---|---|---|
| CH₃ | CH₃ | 180 | 20 | 29 |
| CH₃ | CH₃ | 180 | 30 | 42 |

| | | | | |
|---|---|---|---|---|
| ^{a} As measured by Radio-HPLC. | | | | |

## Claims

1. A method for the preparation of a ¹⁸F-labeled aniline derivative of the general formula I 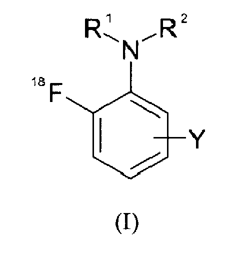 by fluorination of an aniline derivative of the general formula II 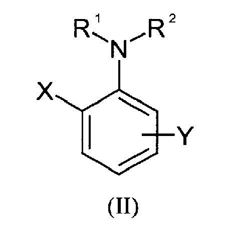 with [¹⁸F]fluoride under the application of microwave heating,
wherein
X is a leaving group;
R¹ and R², independently of one another, are H, alkyl, alkenyl, alkinyl, aryl, heteroaryl and alkylaryl, and wherein R¹ and R² can be joined together to form a ring structure; and
Y represents one or more substituents selected from the group consisting of H, alkyl, alkenyl, alkinyl, aryl, heteroaryl and alkylaryl, halogen, fused aryl and fused heteroaryl.

2. The method according to claim 1, wherein X is selected from the group consisting of halogen atoms (chlorine, bromine, iodine), tosylsulfonate, trifluoromethanesulfonate, tolylsulfonate, triflate, tosylate, trialkyl ammonium, nitro and azide.

3. The method according to any of the preceding claims, wherein the fluorination is performed in a solvent, preferably an aprotic dipolar solvent or an aprotic polar solvent, or mixtures thereof.

4. The method according to claim 3, wherein the solvent is selected from the group consisting of dimethylformamide (DMF), tetrahydrofurane, (THF), dimethylsulfoxide (DMSO), acetonitrile, acetamide and dimethylacetamide (DMAA).

5. The method according to any of the preceding claims, wherein the fluorination is performed at a temperature in the range of from ambient temperature-300°C, preferably in the range of 150-200°C, more preferably in the range of 160-190°C and most preferably in the range of 175-185°C.

6. The method according to any of the preceding claims, wherein the molar ratio of the ¹⁸F-fluoride to the aniline derivative is in the range of 1:1,000-1,000,000, preferably in the range of 1:1,000-500,000.

7. The method according to any of the preceding claims, wherein aniline or an aniline derivative of the general formula II is selected from any of the following formulae 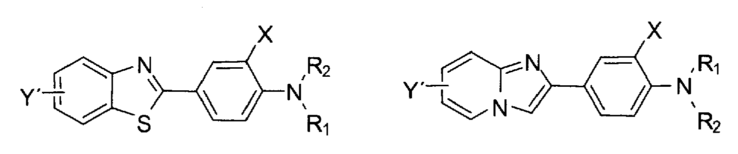 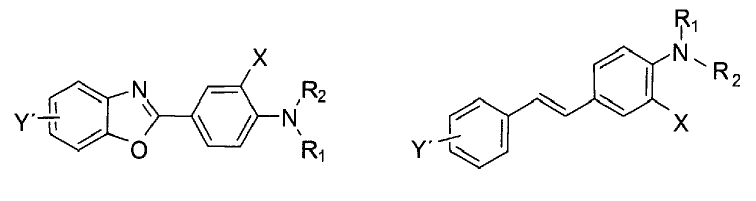 wherein X, R₁ and R₂ are defined as in claim 1 and Y' is defined as Y in claim 1.

8. The method according to any of the preceding claims, wherein R₁ and R₂ are, independently of one another, substituted or unsubstituted, linear, branched or cyclic alkyl, alkenyl or alkinyl, optionally interrupted by one or more heteroatoms, preferably substituted or unsubstituted, linear or branched C₁-C₈ alkyl, C₂-C₈ alkenyl and C₂-C₈ alkinyl, optionally interrupted by one or more heteroatoms, more preferably substituted or unsubstituted, linear or branched C₁-C₆ alkyl, C₂-C₆ alkenyl and C₂-C₆ alkinyl, optionally interrupted by one or more heteroatoms, and most preferably substituted or unsubstituted, linear or branched C₁-C₄ alkyl, C₂-C₄ alkenyl and C₂-C₄ alkinyl, optionally interrupted by one or more heteroatoms.

9. The method according to any of the preceding claims, wherein R₁ and R₂ are, independently of one another, H, methyl or ethyl.

10. The method according to any of the preceding claims, wherein the [¹⁸F]fluoride is used in the fluorination reaction in the presence of a phase transfer catalyst, preferably a tetraalkyl ammonium salt, more preferably a tetrabutyl ammonium salt, or a cryptand, preferably N[CH₂CH₂OCH₂CH₂OCH₂CH₂]₃N, in the presence of potassium carbonate, termed [K/2.2.2]⁺ cryptate.

11. An aniline derivative according to the general formula I 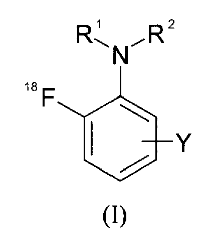 wherein
R¹ and R², independently of one another, are H, alkyl, alkenyl, alkinyl, aryl, heteroaryl and alkylaryl, and wherein R¹ and R² can be joined together to form a ring structure; and
Y represents one or more substituents selected from the group consisting of H, alkyl, alkenyl, alkinyl, aryl, hetaryl, alkylaryl, alkenylaryl, halogen, fused aryl and fused heteroaryl.

12. Use of an aniline derivative according to claim 11 for the preparation of a diagnostic compound for the use in a non-invasive imaging of a mammal, preferably a human, by positron emission tomography.

13. Use according to claim 12, wherein the imaging is carried out to obtain information on the extend, kinetics or treatment response of a disease selected from the group consisting of Alzheimer's disease, mild cognitive impairment syndrome, Parkinson's disease, Lewy body dementia, Down's syndrome and/or frontotemporal degeneration.
